# EUROPEAN PATENT SPECIFICATION

(11) **EP 3 585 411 B1**
(45) Date of publication and mention of the grant of the patent: **28.05.2025**
(21) Application number: 18757393.6
(22) Date of filing: 21.02.2018
(51) Int. Cl.: A61K 38/00, A61K 38/16, A61K 38/20, A61K 39/00

(54) **IL-22BP COMPOSITIONS AND METHOD FOR THE TREATMENT OF DISEASE THEREWITH**
IL-22BP-ZUSAMMENSETZUNGEN UND VERFAHREN ZUR BEHANDLUNG VON KRANKHEITEN DAMIT
COMPOSITIONS À BASE D'IL-22BP ET MÉTHODE POUR LE TRAITEMENT DE MALADIE À L'AIDE DE CELLES-CI

(30) Priority: 21.02.2017 US 201762461543 P
(43) Date of publication of application: 01.01.2020
(73) Proprietor: The Regents of The University of Michigan, Ann Arbor, Michigan 48109 (US)
(72) Inventor: FRANKEL, Timothy, Ann Arbor, Michigan 48109 (US)
(74) Representative: Glawe, Delfs, Moll
(86) International application number: PCT/US2018/018981
(87) International publication number: WO 2018/156602

(56) References cited:
- WO-A2-2005/000897
- WO-A2-2009/023270
- US-A1- 2006 194 735
- US-B2- 7 052 686
- US-B2- 7 094 570
- US-B2- 7 094 570
- WEBER G F ET AL: "IL-22-BP modulates the innate immune response during acute polymicrobial sepsis", IMMUNOBIOLOGY, URBAN UND FISCHER VERLAG, DE, vol. 210, no. 6-8, 1 January 2005 (2005-01-01), pages 496, XP009164364, ISSN: 0171-2985
- PERUSINA LANFRANCA MIRNA ET AL: "Biological and pathological activities of interleukin-22", JOURNAL OF MOLECULAR MEDICINE, SPRINGER VERLAG, DE, vol. 94, no. 5, 29 February 2016 (2016-02-29), pages 523 - 534, XP035801372, ISSN: 0946-2716, [retrieved on 20160229], DOI: 10.1007/S00109-016-1391-6
- MULLER ET AL.: "Improved Pharmacokinetics of Recombinant Bispecific Antibody Molecules by Fusion to Human Serum Albumin", THE JOURNAL OF BIOLOGICAL CHEMISTRY, vol. 282, no. 17, 27 April 2007 (2007-04-27), pages 12650 - 12660, XP055537278

## Description

### CROSS-REFERENCE TO RELATED APPLICATIONS

The present invention claims priority to U.S. Provisional Patent Application Serial Number 62/461,543, filed February 21, 2017.

### FIELD

The invention is set out in the appended set of claims. Provided herein are a fusion protein comprising interleukin-22 binding protein (IL-22BP) and an albumin, a pharmaceutical composition comprising said fusion protein and a pharmaceutically acceptable carrier, and said pharmaceutical composition for use in the treatment or prevention of a disease (e.g. cancer, including pancreatic cancer, e.g. pancreas adenocarcinoma).

### BACKGROUND

Pancreas adenocarcinoma (PDAC) remains among the most lethal cancers with an expected 5-year survival of <5%. Although much is known about the genetic mutations contributing to formation of pre-malignant lesions (PanIN), the initiating events that lead to invasion and dissemination are poorly understood. It is well-established that chronic and familial pancreatitis greatly increase the risk of PDAC (refs. 1-3).

IL-22 is an α-helical cytokine. IL-22 binds to the heterodimeric cell surface receptor, IL-22R, which is composed of IL-10R2 and IL-22R1 subunits (Jones et. al. (2008). Structure. 16 (9): 1333-44). IL-22R is expressed on tissue cells.

### SUMMARY

The invention is set out in the appended set of claims. Provided herein are a fusion protein comprising interleukin-22 binding protein (IL-22BP) and an albumin, a pharmaceutical composition comprising said fusion protein and a pharmaceutically acceptable carrier, and said pharmaceutical composition for use in the treatment or prevention of a disease (e.g. cancer, including pancreatic cancer, e.g. pancreas adenocarcinoma)..

Described herein are compositions comprising an IL-22BP polypeptide and a modifier element (e.g., fused or conjugated together). The IL-22BP polypeptide may have at least 70% (e.g., 70%, 75%, 80%, 85%, 90%, 95%, 96%, 97%, 98%, 99%, 100%, or ranges therebetween) sequence similarity with one or more of SEQ ID NOS: 1-3.

As defined in claim 1, the claimed fusion protein comprises an IL-22BP polypeptide and an albumin. As defined in claim 3, the fusion exhibits enhanced serum half-life relative to full-length wild-type IL-22BP (e.g., half-life enhancement of 10%, 20%, 30%, 40%, 50%, 60%, 70%, 80%, 90%, 2-fold, 3-fold, 4-fold, 5-fold, 6-fold, 7-fold, 8-fold, 9-fold, 10-fold, 20-fold, 50-fold, 100-fold, or more, or ranges therebetween). As defined in claim 4, the fusion is at least twice (e.g., 2x, 3x, 4x, 5x, 6x, 7x, 8x, 9x, 10x, or more, or ranges therebetween) the molecular weight of full-length wild-type IL-22BP. The modifier peptide or polypeptide may be biostable and biocompatible. The fusion may be biostable and biocompatible. As defined in claim 1, the fusion protein comprises albumin.. As defined in claim 5, the albumin comprise at least 70% (e.g., 70%, 75%, 80%, 85%, 90%, 95%, 96%, 97%, 98%, 99%, 100%, or ranges therebetween) sequence identity to SEQ ID NO: 4 or a portion thereof. As defined in claim 6, the IL-22BP polypeptide and the albumin are connected directly, by a peptide linker, or by a non-peptide linker.

As defined in claim 7, provided herein are pharmaceutical compositions comprising the fusion protein of one of claims 1-6 and a pharmaceutically acceptable carrier.

As defined in claim 8, provided herein are a pharmaceutical composition of claim 7 for use in the treatment or prevention of a disease (e.g., cancer (e.g., PDAC)). As defined in claim 9, the disease is cancer. As defined in claim 10, the disease is pancreatic cancer. As defined in claim 11, the disease is pancreas adenocarcinoma (PDAC).

### BRIEF DESCRIPTION OF THE DRAWINGS

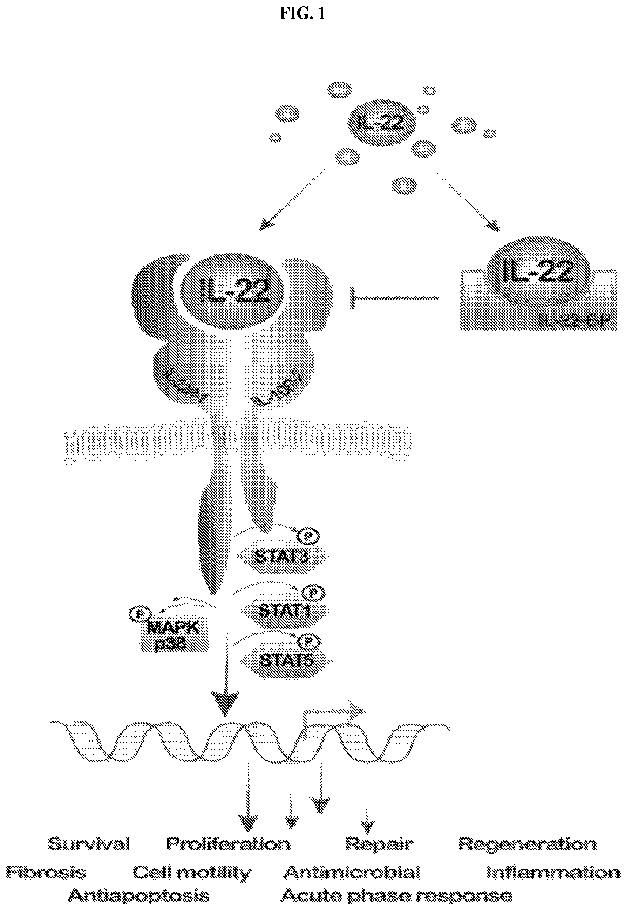

Figure 1. A schematic depicting sequestration of IL-22 using IL-22BP abrogating the pro-tumorigenic effects of IL-22.

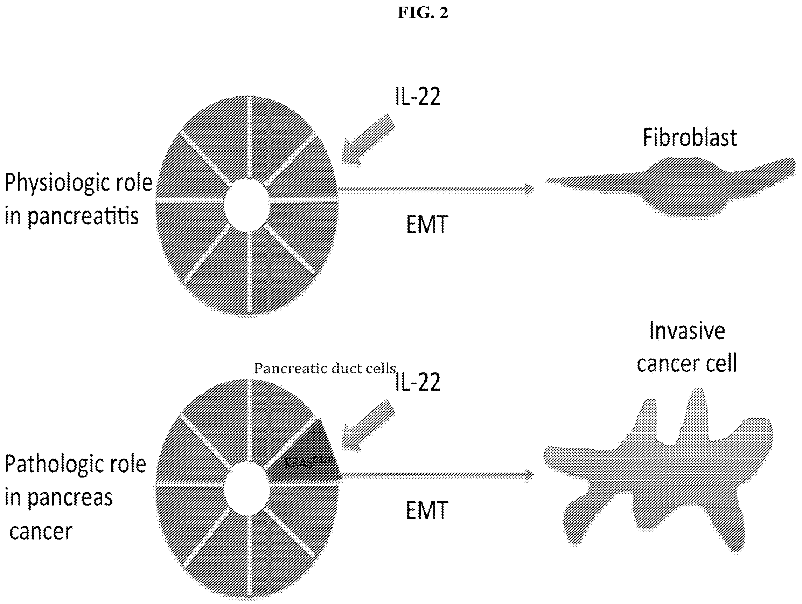

Figure 2. A schematic depicting the physiologic role of IL-22 mediated activation of epithelial-mesenchymal transition (EMT) in pancreatitis and promotion of invasion of genetically transformed cells.
Figure 3A-D. (A) IL-22 is increased following induction of pancreatitis. (B) Immunoblotting reveals abundant IL-22 in surgically resected tumors but not cell lines. (C) Increased IL-22 mRNA is present in tumors, but not (D) IL-2.
Figure 4A-D. (A) Immunoblotting confirms IL-22 dependent activation of STAT3 and NF-κB in human and murine pancreas cancer. IHC for pSTAT3 shows increased intensity from (B) low-grade PanIN to (C) high-grade PanIN and (D) invasive cancer.
Figure 5A-D. Matrigel invasion assay confirms that IL-22 promotes invasion of (A) PDAC and (B) PanIN cell lines. *In-vivo,* SQ tumor tumors developed more frequently (C) and were larger (D) in wild-type mice compared to IL-22 knockout mice.
Figure 6A-E. IL-22 decreases cisplatin mediated cell death (A) by decreasing DNA damage mediate apoptosis (B). hIL-22BP is capable of binding and neutralizing both human (C) and mouse (D) IL-22. A fusion protein of hIL-22BP and albumin shows similar efficacy (E).
Figure 7. exemplary IL-22BP-Alb constructs.

### DEFINITIONS

The following terminology is used in the description:
Unless otherwise defined, all technical and scientific terms used herein have the same meaning as commonly understood by one of ordinary skill in the art to which this invention belongs. However, in case of conflict, the present specification, including definitions, will control. Accordingly, in the context of the present disclosure, the following definitions apply.

As used herein and in the appended claims, the singular forms "a", "an" and "the" include plural reference unless the context clearly dictates otherwise. Thus, for example, reference to "an IL-22BP fusion protein" is a reference to one or more IL-22BP fusion proteins and equivalents thereof known to those skilled in the art, and so forth.

As used herein, the term "comprise" and linguistic variations thereof denote the presence of recited feature(s), element(s), method step(s), etc. without the exclusion of the presence of additional feature(s), element(s), method step(s), etc. Conversely, the term "consisting of" and linguistic variations thereof, denotes the presence of recited feature(s), element(s), method step(s), etc. and excludes any unrecited feature(s), element(s), method step(s), etc., except for ordinarily-associated impurities. The phrase "consisting essentially of" denotes the recited feature(s), element(s), method step(s), etc. and any additional feature(s), element(s), method step(s), etc. that do not materially affect the basic nature of the composition, system, or method. Many embodiments herein are described using open "comprising" language. Such embodiments encompass multiple closed "consisting of" and/or "consisting essentially of" embodiments, which may alternatively be claimed or described using such language.

As used herein, the term "pharmaceutically acceptable carrier" refers to non-toxic solid, semisolid, or liquid filler, diluent, encapsulating material, formulation auxiliary, or carrier conventional in the art for use with a therapeutic agent for administration to a subject. A pharmaceutically acceptable carrier is non-toxic to recipients at the dosages and concentrations employed and is compatible with other ingredients of the formulation. The pharmaceutically acceptable carrier is appropriate for the formulation employed. For example, if the therapeutic agent is to be administered orally, the carrier may be a gel capsule. A "pharmaceutical composition" typically comprises at least one active agent (e.g., the copolymers described herein) and a pharmaceutically acceptable carrier.

As used herein, the term "effective amount" refers to the amount of a composition (e.g., pharmaceutical composition) sufficient to effect beneficial or desired results. An effective amount can be administered in one or more administrations, applications or dosages and is not intended to be limited to a particular formulation or administration route.

As used herein, the term "administration" refers to the act of giving a drug, prodrug, or other agent, or therapeutic treatment (e.g., pharmaceutical compositions of the present invention) to a subject or *in vivo, in vitro,* or *ex vivo* cells, tissues, and organs. Exemplary routes of administration to the human body can be through the eyes (e.g., intraocularly, intravitrealy, periocularly, ophthalmic, etc.), mouth (oral), skin (transdermal), nose (nasal), lungs (inhalant), oral mucosa (buccal), ear, rectal, by injection (e.g., intravenously, subcutaneously, intratumorally, intraperitoneally, etc.) and the like.

As used herein, the terms "co-administration" and "co-administer" refer to the administration of at least two agent(s) or therapies to a subject. The co-administration of two or more agents or therapies may be concurrent (e.g., in the same or separate formulations) or a first agent/therapy is administered prior to a second agent/therapy. Those of skill in the art understand that the formulations and/or routes of administration of the various agents or therapies used may vary. The appropriate dosage for co-administration can be readily determined by one skilled in the art. When agents or therapies are co-administered, the respective agents or therapies are administered at lower dosages than appropriate for their administration alone. Thus, co-administration is especially desirable where the co-administration of the agents or therapies lowers the requisite dosage of a potentially harmful (e.g., toxic) agent(s).

The term "amino acid" refers to natural amino acids, unnatural amino acids, and amino acid analogs, all in their D and L stereoisomers, unless otherwise indicated, if their structures allow such stereoisomeric forms.

Natural amino acids include alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), Lysine (Lys or K), methionine (Met or M), phenylalanine (Phe or F), proline (Pro or P), serine (Ser or S), threonine (Thr or T), tryptophan (Trp or W), tyrosine (Tyr or Y) and valine (Val or V).

Unnatural amino acids include, but are not limited to, azetidinecarboxylic acid, 2-aminoadipic acid, 3-aminoadipic acid, beta-alanine, naphthylalanine ("naph"), aminopropionic acid, 2-aminobutyric acid, 4-aminobutyric acid, 6-aminocaproic acid, 2-aminoheptanoic acid, 2-aminoisobutyric acid, 3-aminoisbutyric acid, 2-aminopimelic acid, tertiary-butylglycine ("tBuG"), 2,4-diaminoisobutyric acid, desmosine, 2,2'-diaminopimelic acid, 2,3-diaminopropionic acid, N-ethylglycine, N-ethylasparagine, homoproline ("hPro" or "homoP"), hydroxylysine, allo-hydroxylysine, 3-hydroxyproline ("3Hyp"), 4-hydroxyproline ("4Hyp"), isodesmosine, allo-isoleucine, N-methylalanine ("MeAla" or "Nime"), N-alkylglycine ("NAG") including N-methylglycine, N-methylisoleucine, N-alkylpentylglycine ("NAPG") including N-methylpentylglycine. N-methylvaline, naphthylalanine, norvaline ("Norval"), norleucine ("Norleu"), octylglycine ("OctG"), ornithine ("Orn"), pentylglycine ("pG" or "PGly"), pipecolic acid, thioproline ("ThioP" or "tPro"), homoLysine ("hLys"), and homoArginine ("hArg").

The term "amino acid analog" refers to a natural or unnatural amino acid where one or more of the C-terminal carboxy group, the N-terminal amino group and side-chain functional group has been chemically blocked, reversibly or irreversibly, or otherwise modified to another functional group. For example, aspartic acid-(beta-methyl ester) is an amino acid analog of aspartic acid; N-ethylglycine is an amino acid analog of glycine; or alanine carboxamide is an amino acid analog of alanine. Other amino acid analogs include methionine sulfoxide, methionine sulfone, S-(carboxymethyl)-cysteine, S-(carboxymethyl)-cysteine sulfoxide and S-(carboxymethyl)-cysteine sulfone.

As used herein, the term "peptide" refers a short polymer of amino acids linked together by peptide bonds. In contrast to other amino acid polymers (e.g., proteins, polypeptides, etc.), peptides are of about 50 amino acids or less in length. A peptide may comprise natural amino acids, non-natural amino acids, amino acid analogs, and/or modified amino acids. A peptide may be a subsequence of naturally occurring protein or a non-natural (synthetic) sequence.

As used herein, the term "mutant", when used in reference to a peptide/polypeptide/protein, refers to an amino acid sequence that is distinct from the most common variant occurring in nature, referred to as the "wild-type" sequence. A mutant peptide/polypeptide may be a subsequence of a mutant protein or polypeptide (e.g., a subsequence of a naturally-occurring protein that is not the most common sequence in nature) or may be a peptide/polypeptide that is not a subsequence of a naturally occurring protein or polypeptide.

As used herein, the term "artificial" refers to a peptide or polypeptide having a distinct amino acid sequence from those found in natural peptides and/or proteins. An artificial protein is not a subsequence of a naturally occurring protein, either the wild-type (i.e., most abundant) or mutant versions thereof. For example, an artificial IL-22BP polypeptide is not a subsequence of naturally occurring IL-22BP. An artificial peptide or polypeptide may be produced or synthesized by any suitable method (e.g., recombinant expression, chemical synthesis, enzymatic synthesis, etc.).

The term "peptidomimetic" refer to a peptide-like or polypeptide-like molecule that emulates a sequence derived from a protein or peptide. A peptidomimetic may contain amino acids and/or non-amino acid components. Examples of peptidomimitecs include chemically modified peptides/polypeptides, peptoids (side chains are appended to the nitrogen atom of the peptide backbone, rather than to the α-carbons), β-peptides (amino group bonded to the β carbon rather than the α carbon), etc.

As used herein, a "conservative" amino acid substitution refers to the substitution of an amino acid in a peptide or polypeptide with another amino acid having similar chemical properties, such as size or charge. For purposes of the present disclosure, each of the following eight groups contains amino acids that are conservative substitutions for one another:
1) Alanine (A) and Glycine (G);
2) Aspartic acid (D) and Glutamic acid (E);
3) Asparagine (N) and Glutamine (Q);
4) Arginine (R) and Lysine (K);
5) Isoleucine (I), Leucine (L), Methionine (M), and Valine (V);
6) Phenylalanine (F), Tyrosine (Y), and Tryptophan (W);
7) Serine (S) and Threonine (T); and
8) Cysteine (C) and Methionine (M).

Naturally occurring residues may be divided into classes based on common side chain properties, for example: polar positive (histidine (H), lysine (K), and arginine (R)); polar negative (aspartic acid (D), glutamic acid (E)); polar neutral (serine (S), threonine (T), asparagine (N), glutamine (Q)); non-polar aliphatic (alanine (A), valine (V), leucine (L), isoleucine (I), methionine (M)); non-polar aromatic (phenylalanine (F), tyrosine (Y), tryptophan (W)); proline and glycine; and cysteine. As used herein, a "semi-conservative" amino acid substitution refers to the substitution of an amino acid in a peptide or polypeptide with another amino acid within the same class.

Uunless otherwise specified, a conservative or semi-conservative amino acid substitution may also encompass non-naturally occurring amino acid residues that have similar chemical properties to the natural residue. These non-natural residues are typically incorporated by chemical peptide synthesis rather than by synthesis in biological systems. These include, but are not limited to, peptidomimetics and other reversed or inverted forms of amino acid moieties. There might be a limitation to natural amino acids, non-natural amino acids, and/or amino acid analogs.

Non-conservative substitutions may involve the exchange of a member of one class for a member from another class.

As used herein, the term "sequence identity" refers to the degree to which two polymer sequences (e.g., peptide, polypeptide, nucleic acid, etc.) have the same sequential composition of monomer subunits. The term "sequence similarity" refers to the degree with which two polymer sequences (e.g., peptide, polypeptide, nucleic acid, etc.) differ only by conservative and/or semi-conservative amino acid substitutions. The "percent sequence identity" (or "percent sequence similarity") is calculated by: (1) comparing two optimally aligned sequences over a window of comparison (e.g., the length of the longer sequence, the length of the shorter sequence, a specified window, etc.), (2) determining the number of positions containing identical (or similar) monomers (e.g., same amino acids occurs in both sequences, similar amino acid occurs in both sequences) to yield the number of matched positions, (3) dividing the number of matched positions by the total number of positions in the comparison window (e.g., the length of the longer sequence, the length of the shorter sequence, a specified window), and (4) multiplying the result by 100 to yield the percent sequence identity or percent sequence similarity. For example, if peptides A and B are both 20 amino acids in length and have identical amino acids at all but 1 position, then peptide A and peptide B have 95% sequence identity. If the amino acids at the non-identical position shared the same biophysical characteristics (e.g., both were acidic), then peptide A and peptide B would have 100% sequence similarity. As another example, if peptide C is 20 amino acids in length and peptide D is 15 amino acids in length, and 14 out of 15 amino acids in peptide D are identical to those of a portion of peptide C, then peptides C and D have 70% sequence identity, but peptide D has 93.3% sequence identity to an optimal comparison window of peptide C. For the purpose of calculating "percent sequence identity" (or "percent sequence similarity") herein, any gaps in aligned sequences are treated as mismatches at that position.

As used herein, the terms "conjugate" (and linguistic variations thereof, such as, "conjugated") refer to a construct in which protein, peptide, or polypeptide component is attached to a second component (e.g., a modifier). A conjugate may comprise a protein of interest (e.g., IL-22BP) attached to any suitable non-peptide/non-polypeptide modifier molecule (e.g., a linear or branched polymer, a lipid; a cholesterol group (such as a steroid); a carbohydrate or oligosaccharide, a small molecule, etc.). The peptide/polypeptide component of the conjugate may be expressed/synthesized by any suitable routes, but the non-peptide/non-polypeptide component is typically attached, chemically and/or enzymatically, after expression/synthesis of the peptide/polypeptide component. Alternatively, a conjugate may comprise a protein of interest (e.g., IL-22BP) attached to any suitable peptide/polypeptide modifier; such conjugates are referred to herein as "fusions."

As used herein, the terms "fusion" and "fusion protein" (and linguistic variations thereof, such as, "fused") refer to a protein or polypeptide construct (a conjugate) comprising peptide/polypeptide components (e.g., IL-22BP and a modifier peptide/polypeptide) derived from more than one parental protein or polypeptide (e.g., or natural or artificial origin). A fusion protein may be expressed from a fusion gene in which a nucleotide sequence encoding a polypeptide sequence from one peptide/polypeptide is appended in frame with, and optionally separated by a linker from, a nucleotide sequence encoding a peptide/polypeptide sequence from a different protein.

As used herein, the term "modifier" refers to a molecule that, when fused or conjugated to a therapeutic protein, polypeptide or peptide, prevents clearance, prevents degradation, increases plasma half-life, reduces toxicity, reduces immunogenicity, or increases biological activity of a therapeutic protein, polypeptide, or peptide. Exemplary modifiers include, but are not limited to, albumin, an Fc domain (e.g., of IgG), a linear polymer (e.g., polyethylene glycol (PEG), polylysine, dextran, etc.), a branched-chain polymer (see, for example, U.S. Pat. No. 4,289,872, U.S. Pat. No. 5, 229,490; WO 93/21259); a lipid; a cholesterol group (such as a steroid); a carbohydrate or oligosaccharide; or a natural or synthetic protein, polypeptide or peptide that binds to a salvage receptor.

As used herein, the term "biocompatible" refers to materials, compounds, or compositions that do not cause or elicit significant adverse effects when administered to a subject. Examples of possible adverse effects that limit biocompatibility include, but are not limited to, excessive or adverse immune response, and toxicity.

As used herein, the term "biostable" refers to compositions or materials that do not readily break-down or degrade in a physiological or similar aqueous environment. Conversely, the term "biodegradeable" refers herein to compositions or materials that readily decompose (e.g., depolymerize, hydrolyze, are enzymatically degraded, disassociate, etc.) in a physiological or other environment.

As used herein, the term "pharmaceutically acceptable carrier" refers to non-toxic solid, semisolid, or liquid filler, diluent, encapsulating material, formulation auxiliary, or carrier conventional in the art for use with a therapeutic agent for administration to a subject. A pharmaceutically acceptable carrier is non-toxic to recipients at the dosages and concentrations employed and is compatible with other ingredients of the formulation. The pharmaceutically acceptable carrier is appropriate for the formulation employed. For example, if the therapeutic agent is to be administered orally, the carrier may be a gel capsule. A "pharmaceutical composition" typically comprises at least one active agent (e.g., the copolymers described herein) and a pharmaceutically acceptable carrier.

As used herein, the term "effective amount" refers to the amount of a composition (e.g., pharmaceutical composition) sufficient to effect beneficial or desired results. An effective amount can be administered in one or more administrations, applications or dosages and is not intended to be limited to a particular formulation or administration route.

As used herein, the term "administration" refers to the act of giving a drug, prodrug, or other agent, or therapeutic treatment (e.g., pharmaceutical compositions of the present invention) to a subject or in vivo, in vitro, or ex vivo cells, tissues, and organs. Exemplary routes of administration to the human body can be through the eyes (e.g., intraocularly, intravitrealy, periocularly, ophthalmic, etc.), mouth (oral), skin (transdermal), nose (nasal), lungs (inhalant), oral mucosa (buccal), ear, rectal, by injection (e.g., intravenously, subcutaneously, intratumorally, intraperitoneally, etc.) and the like.

As used herein, the terms "co-administration" and "co-administer" refer to the administration of at least two agent(s) or therapies to a subject. The co-administration of two or more agents or therapies may be concurrent (e.g., in the same or separate formulations) or a first agent/therapy is administered prior to a second agent/therapy. Those of skill in the art understand that the formulations and/or routes of administration of the various agents or therapies used may vary. The appropriate dosage for co-administration can be readily determined by one skilled in the art. When agents or therapies are co-administered, the respective agents or therapies may be administered at lower dosages than appropriate for their administration alone. Thus, co-administration is especially desirable where the co-administration of the agents or therapies lowers the requisite dosage of a potentially harmful (e.g., toxic) agent(s).

### DETAILED DESCRIPTION

The invention is set out in the appended set of claims. Provided herein are a fusion protein comprising interleukin-22 binding protein (IL-22BP) and an albumin, a pharmaceutical composition comprising said fusion protein and a pharmaceutically acceptable carrier, and said pharmaceutical composition for use in the treatment or prevention of a disease (e.g. cancer, including pancreatic cancer, e.g. pancreas adenocarcinoma).

IL-22R1 is the receptor of IL-22, which is found only on epithelial cells, positioning IL-22 as a mediator of immune-epithelial cancer interactions. Signaling leads to activation of STAT3 and nuclear factor kappa B (NF-κB) pathways which have been previously linked to transcription of pro-tumorigenic factors (ref. 10-13). Pancreatic ductal cells possess the highest IL-22R1 concentration in the body (ref. 14) and IL-22 is critical to organ repair following acute pancreatitis (ref. 15, 16). Data also demonstrate that elevated IL-22 in PDAC is associated with poor survival (ref. 17, 18). In the colon, IL-22 signaling at the tissue level is regulated by the presence of IL-22 binding protein (IL-22BP) a soluble receptor capable of cytokine neutralization.

Experiments have demonstrated: elevated levels of IL-22 and its associated receptor in pancreatitis, PanIN and invasive cancers as well as increased quantities of IL-22 secreting cells; that IL-22 promotes chemoresistance, proliferation and invasion, in-vitro, as well as tumor establishment and growth, *in vivo;* the ability of IL-22BP to block IL-22 mediated STAT3 signaling; and neutralization of the effects of IL-22 using engineered fusions of IL-22BP with carrier proteins. As defined in claims 7 and 8, provided herein are pharmaceutical compositions and the use thereof for the treatment and prevention of disease (e.g., cancer).

IL-22BP has a relatively short circulation half-life in the human body because it is prone to clearance by filtration due to small molecular weight. Extension of *in vivo* half-life of IL-22BP provides reduced dose concentrations, fewer doses, and/or increased efficacy. One route to increase *in vivo* half-life of IL-22BP protein is to decrease *in vivo* clearance of the protein, including clearance through kidney, protease degradation, and receptor-mediated clearance. To achieve this goal, IL-22BP may be conjugated to components (e.g., peptide or protein carriers) that, for example, increase the apparent molecular weight of IL-22BP, so as to slow down the renal clearance rate. Suitable carriers function merely by increasing the molecular weight, without otherwise detrimentally affecting IL-22BP function. Carriers may provide additional functionality to enhance the efficacy of IL-22BP, localize IL-22BP (e.g., to cancer cells, etc.), to enhance stability, etc. For example, a carrier may prevent protease degradation of IL-22BP. As defined in claim 4, provided herein are fusion proteins comprising an IL-22BP polypeptide and albumin that increase at least twice the apparent molecular weight of full length wild-type IL-22BP.

As defined in claim 3 the serum half-life of the fusion protein is enhanced relative to full-length wild-type IL-22BP.

For some proteins having relatively long half-life in serum, such as albumin and IgG, there is an FcRn-mediated protection effect against endocytosis with the basic mechanism that Fc region of IgG and albumin binds to a corresponding FcRn receptor on cell surface under normal physiological conditions, and then are endocytosed after binding. Due to the decreased pH in a phagocyte, the bound complex disassociates, IgG and albumin are released from the cells again. IgG and albumin are protected against degradation and metabolism because of the presence of the FcRn-mediated circulation (Junghans R P, Immunol Res., 16:29-57, 1997; Chaudhury C et al, J Exp Med., 197: 315-322, 2003; Chaudhury C et al, Biochemistry., 45:4983-4990, 2006); therefore, fusion of IL-22BP with albumin, the Fc fragment of IgG, or other similar proteins prolongs IL-22BP half-life.

As defined in the claims. fusions of IL-22BP and/or an IL-22BP-based peptide or polypeptide with albumin are provided.

IL22-BP itself may be modified to enhance biostability and/or biocompatibility, to extend the serum half-life, and/or to prevent/inhibit/reduce clearance (e.g., by the kidneys). Modification may include substitution/deletion/addition of amino acids from wild-type IL-22BP (e.g., SEQ ID NO:1 or SEQ ID NO: 2). One or more positons that are not conserved between human and mouse IL-22BP (SEQ ID NOS:1 and 2), as indicated by X in SEQ ID NO: 3 may be substituted (with respect to SEQ ID NO: 1 and/or 2). An IL-22BP-based polypeptide may comprise one or more substitutions (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, or ranges therebetween) relative to SEQ ID NO: 1 and/or 2. An IL-22BP-based polypeptide may comprise a truncation (or deletion) relative to SEQ ID NO: 1 and/or 2. A truncation (or deletion) may be at the C-terminus, N-terminus, or internally. A truncation (or deletion) may be one or more residues in length (e.g., 1, 2, 3, 4, 5, 6, 7, 8, 9, 10, 15, 20, 25, 30, 35, 40, 45, 50, 55, 60, 65, 70, 75, 80, 85, 90, 95, 100, 110, 120, 130, 140, 150, or ranges therebetween) relative to SEQ ID NO: 1 and/or 2. An IL-22BP-based polypeptide may comprise at least 70% (e.g., 70%, 75%, 80%, 85%, 90%, 95%, 99%, 100%) sequence identity with all or a portion (e.g., 50 residue portion, 60 residue portion, 70 residue portion, 80 residue portion, 90 residue portion, 1000 residue portion, 1100 residue portion, 120 residue portion, 130 residue portion, 140 residue portion, 150 residue portion, 160 residue portion, 170 residue portion, 180 residue portion, 190 residue portion, 200 residue portion, 210 residue portion, 220 residue portion, 230 residue portion, or ranges therebetween). An IL-22BP-based polypeptide may comprise at least 70% (e.g., 70%, 75%, 80%, 85%, 90%, 95%, 99%, 100%) sequence similarity (e.g., conservative or semiconservative) with all or a portion (e.g., 50 residue portion, 60 residue portion, 70 residue portion, 80 residue portion, 90 residue portion, 1000 residue portion, 1100 residue portion, 120 residue portion, 130 residue portion, 140 residue portion, 150 residue portion, 160 residue portion, 170 residue portion, 180 residue portion, 190 residue portion, 200 residue portion, 210 residue portion, 220 residue portion, 230 residue portion, or ranges therebetween). An IL-22BP-based polypeptide may comprise one or more modified or unnatural amino acids. An IL-22BP-based polypeptide may be a peptidomimetic.

An IL-22BP-based polypeptide may bind to IL-22, and may regulate (e.g., inhibits) signaling thereby. A modified IL-22BP provided herein may bind IL-22 with greater affinity than wild-type IL-22BP binds IL-22.

Disclosed herein are fusions of IL-22BP (e.g., SEQ ID NO:1, 2, or 3) and/or an IL-22BP-based polypeptide described above with a modifier peptide or polypeptide. The modifier may be between 10 and 1000 amino acid residues in length (e.g., 10, 20, 30, 40, 50, 60, 70, 80, 90, 100, 150, 200, 250, 300, 350, 400, 450, 500, 550, 600, 650, 700, 750, 800, 850, 900, 950, 1000, or ranges therebetween). The modifier may be biostable and biocompatible. The modifier may extend the half-life and/or may prevent clearance (e.g., by the kidney) of the fusion, relative to IL-22BP alone, due to the extra size/bulk/mass of the fusion. The modifier may possess one or more characteristics that further (e.g., in addition to adding size/bulk/mass) extends the half-life and/or prevents clearance (e.g., by the kidney) of the fusion, relative to IL-22BP alone (e.g., protects against endocytosis). The modifier may be a naturally long-half-life protein or protein domain (e.g., an Fc domain, transferrin (Tf), albumin, etc.). The modifier may be an inert polypeptide (e.g., XTEN (Schellenberger et al. Nat Biotechnol. 2009 Dec;27(12):1186-90), a homo-amino acid polymer (Schlapschy et al. Protein Eng Des Sel. 2007;20:273-284), a proline-alanine-serine polymer (Schlapschy et al. Protein Eng Des Sel. 2013;26:489-501), or an elastin-like peptide (Floss et al. Trends Biotechnol. 2010;28:37-45), etc.). The modifier may be a negatively charged, highly sialylated peptide (e.g., carboxy-terminal peptide (Duijkers et al. Hum Reprod. 2002;17:1987-1993), etc.). A modifier may be a fragment or a variant of transferrin, albumin, an Fc domain, carboxy-terminal peptide, proline-alanine-serine polymer, elastin-like peptide, or XTEN.

As defined in claim 1, provided herein are fusion proteins comprising an IL-22BP polypeptide and an albumin. As defined in claim 5, the fusion protein comprises albumin comprising at least 70% sequence identity (e.g., 70%, 75%, 80%, 85%, 90%, 95%, 99%, 100%, or ranges therebetween) to SEQ ID NO: 4 or a portion thereof.

Disclosed herein are conjugates of IL-22BP (e.g., SEQ ID NO:1, 2, or 3) and/or an IL-22BP-based polypeptide described herein with a non-peptide/non-polypeptide modifier element (e.g., compound, polymer, etc.). The modifier element may be biostable and biocompatible. The modifier element may extend the half-life and/or may prevent clearance (e.g., by the kidney) of the fusion, relative to IL-22BP alone, due to the extra size/bulk/mass of the conjugate. The modifier element may possess one or more characteristics that further (e.g., in addition to adding size/bulk/mass) extends the half-life and/or prevents clearance (e.g., by the kidney) of the fusion, relative to IL-22BP alone (e.g., increases the hydrodynamic radius, etc.). The modifier element may be a polymer, such as PEG, dextran, polysialic acids, hyaluronic acid, dextrin, hydroxyethyl-starch, poly(2-ethyl 2-oxazoline), etc. (Paust. Polymers 2014, 6, 160-178). Conjugate may be a glycosylated IL-22BP.

A IL-22BP-based construct may be provided that extends the half-life (e.g., serum half-life) of IL-22BP or variants thereof and comprises both peptide/polypeptide (e.g., albumin, Fc domain, XTEN, etc.) and non-peptide/non-polypeptide (e.g., PEG, hyaluronic acid, gluycosylation, etc.) modifiers.

The IL-22BP-based polypeptide and the modifier element may be connected directly. The IL-22BP-based polypeptide and the modifier element may be connected (e.g., conjugated, fused, etc.) by a linker. Suitable linkers may be peptide or polypeptide linkers (e.g., connecting polypeptide domains), or may be chemical linkers (e.g., connecting a IL-22BP-based polypeptide to a non-peptide/non-polypeptide modifier). A linker may be of a length and composition to allow the proper folding and/or activity of the IL-22BP-based polypeptide and the modifier element. Linkers may be of any suitable flexibility or rigidity.

A linker may be a non-peptide/non-polypeptide linker. Indeed, a variety of non-peptide/non-polypeptide linkers are contemplated, and suitable linkers could comprise, but are not limited to, alkyl groups, methylene carbon chains, ether, polyether, alkyl amide linker, a peptide linker, a modified peptide linker, a Poly(ethylene glycol) (PEG) linker, a streptavidin-biotin or avidin-biotin linker, polyaminoacids (e.g. polylysine), functionalized PEG, polysaccharides, glycosaminoglycans, dendritic polymers (WO93/06868 and by Tomalia et al. in Angew. Chem. Int. Ed. Engl. 29:138-175 (1990)), PEG-chelant polymers (W94/08629, WO94/09056 and WO96/26754), oligonucleotide linker, phospholipid derivatives, alkenyl chains, alkynyl chains, disulfide, or a combination thereof. A linker may comprise any combination of alkyl, alkenyl, alkynyl, phenyl, benzyl, halo, fluoro, chloro, bromo, bromo, iodo, hydroxyl, carbonyl, aldehyde, haloformyl, carbonate ester, carboxylate, carboxyl, ester, hydroperoxy, peroxy, ether, hemiacetal, hemiketal, acetal, ketal, orthoester, amide, amine, imine, imide, azide, azo, cyanate, nitrate, nitrite, nitrile, nitro, nitroso, pyridine, thiol, sulfide, disulfide, sulfoxide, sulfone, sulifinic acid, sulfonic acid, thiocyanate, thione, thial, phosphine, phosphonic acid, phosphate, and/or phosphodiester groups.

A linker may be a peptide linker (e.g., when the conjugate is a fusion). A linker peptide may comprise one or more additional functionalities, in addition to appropriately-connecting the IL-22BP-based polypeptide and the modifier element. For example, the linker segment may comprise a cleavable peptide which allows the IL-22BP-based polypeptide and the modifier element to be separated from one another using appropriate conditions. The linker peptide may comprise a protease recognition amino acid sequence specifically recognized as a cleavage site by a protease selected from the group consisting of a serine protease, a threonine protease, a cysteine protease, an aspartic acid protease, a matrix metalloproteinase, and a glutamic acid protease. The peptide linker may comprise a site-specific protease recognition amino acid sequence specifically recognized as a cleavage substrate by a protease selected from the group consisting of furan protease, tobacco etch virus ("TEV") protease, a 3C protease, a caspase, a matrix metalloproteinase, etc. A linker may be a rigid peptide (e.g., SEQ ID NO: 5). A linker may be a rigid peptide (e.g., SEQ ID NO: 6).

As defined in the claims, the pharmaceutical composition comprising the fusion protein comprising IL-22BP polypeptide and albumin find use in the treatment and/or prevention of a disease, including a wide range of diseases and conditions, such as, autoimmune diseases, allergies (e.g., food allergies), inflammatory conditions, metabolic disorders, cancer, diabetes, etc. Said pharmaceutical compositions may find use in the prevention and/or treatment of inflammatory bowel disease, irritable bowel syndrome, cancer, multiple sclerosis, Alzheimer's disease, rheumatoid arthritis, coeliac disease, diabetes mellitus, psoriasis, etc. As defined in claim 9, the pharmaceutical compositions described herein find use in the treatment and/or prevention of cancer. Non-limiting examples of cancers that may be treated with the pharmaceutical compositions include, but are not limited to: melanoma (e.g., metastatic malignant melanoma), renal cancer (e.g. clear cell carcinoma), prostate cancer (e.g. hormone refractory prostate adenocarcinoma), pancreatic cancer (e.g., adenocarcinoma), breast cancer, colon cancer, lung cancer (e.g. non-small cell lung cancer), esophageal cancer, squamous cell carcinoma of the head and neck, liver cancer, ovarian cancer, cervical cancer, thyroid cancer, glioblastoma, glioma, leukemia, lymphoma, and other neoplastic malignancies. The cancer may be a solid tumor cancer.

The fusion protein as defined in claims 1-6 are provided as pharmaceutical compositions as defined in claim 7. Pharmaceutical preparations can be formulated from the compositions herein by drug formulation methods known to those skilled in the art. Formulations are prepared using a pharmaceutically acceptable carrier composed of materials that are considered safe and effective, without causing undesirable biological side effects or unwanted interactions. Suitable carriers include, but are not limited to, saline, buffered saline, dextrose, water, glycerol, ethanol, and combinations thereof. The composition can be adapted for the mode of administration and can be in the form of, e.g., a pill, tablet, capsule, spray, powder, or liquid. The pharmaceutical composition may contain one or more pharmaceutically acceptable additives suitable for the selected route and mode of administration, such as coatings, fillers, binders, lubricant, disintegrants, stabilizers, or surfactants. These compositions may be administered by, without limitation, any parenteral route, including intravenous, intra-arterial, intramuscular, subcutaneous, intradermal, intraperitoneal, intrathecal, as well as topically, orally, and by mucosal routes of delivery such as intranasal, inhalation, rectal, vaginal, buccal, and sublingual. The pharmaceutical compositions of the invention as defined in the claims may be prepared for administration to vertebrate (e.g. mammalian e.g., human)) subjects in the form of liquids, including sterile, non-pyrogenic liquids for injection, emulsions, powders, aerosols, tablets, capsules, enteric coated tablets, or suppositories.

Method of treatments covered by Article 53(c) EPC are not comprised by the invention. Therefore, the following aspects are outlined for information purposes only: A subject may be treated with (i) an IL-22BP-based construct, as described herein (e.g., conjugate or fusion), as well as (ii) one or more additional cancer therapies. Such therapies include chemotherapy, immunotherapy, radiation, surgery, etc. Exemplary anticancer agents suitable for use in compositions and methods described herein include, but are not limited to: 1) alkaloids, including microtubule inhibitors (e.g., vincristine, vinblastine, and vindesine, etc.), microtubule stabilizers (e.g., paclitaxel (Taxol), and docetaxel, etc.), and chromatin function inhibitors, including topoisomerase inhibitors, such as epipodophyllotoxins (e.g., etoposide (VP-16), and teniposide (VM-26), etc.), and agents that target topoisomerase I (e.g., camptothecin and isirinotecan (CPT-11), etc.); 2) covalent DNA-binding agents (alkylating agents), including nitrogen mustards (e.g., mechlorethamine, chlorambucil, cyclophosphamide, ifosphamide, and busulfan (MYLERAN), etc.), nitrosoureas (e.g., carmustine, lomustine, and semustine, etc.), and other alkylating agents (e.g., dacarbazine, hydroxymethylmelamine, thiotepa, and mitomycin, etc.); 3) noncovalent DNA-binding agents (antitumor antibiotics), including nucleic acid inhibitors (e.g., dactinomycin (actinomycin D), etc.), anthracyclines (e.g., daunorubicin (daunomycin, and cerubidine), doxorubicin (adriamycin), and idarubicin (idamycin), etc.), anthracenediones (e.g., anthracycline analogues, such as mitoxantrone, etc.), bleomycins (BLENOXANE), etc., and plicamycin (mithramycin), etc.; 4) antimetabolites, including antifolates (e.g., methotrexate, FOLEX, and MEXATE, etc.), purine antimetabolites (e.g., 6-mercaptopurine (6-MP, PURINETHOL), 6-thioguanine (6-TG), azathioprine, acyclovir, ganciclovir, chlorodeoxyadenosine, 2-chlorodeoxyadenosine (CdA), and 2'-deoxycoformycin (pentostatin), etc.), pyrimidine antagonists (e.g., fluoropyrimidines (e.g., 5-fluorouracil (ADRUCIL), 5-fluorodeoxyuridine (FdUrd) (floxuridine)) etc.), and cytosine arabinosides (e.g., CYTOSAR (ara-C) and fludarabine, etc.); 5) enzymes, including L-asparaginase, and hydroxyurea, etc.; 6) hormones, including glucocorticoids, antiestrogens (e.g., tamoxifen, etc.), nonsteroidal antiandrogens (e.g., flutamide, etc.), and aromatase inhibitors (e.g., anastrozole (ARIMIDEX), etc.); 7) platinum compounds (e.g., cisplatin and carboplatin, etc.); 8) monoclonal antibodies (e.g., conjugated with anticancer drugs, toxins, and/or radionuclides, etc.; neutralizing antibodies; etc.); 9) biological response modifiers (e.g., interferons (e.g., IFN-.alpha., etc.) and interleukins (e.g., IL-2, etc.), etc.); 10) adoptive immunotherapy; 11) hematopoietic growth factors; 12) agents that induce tumor cell differentiation (e.g., all-trans-retinoic acid, etc.); 13) gene therapy techniques; 14) antisense therapy techniques; 15) tumor vaccines; 16) therapies directed against tumor metastases (e.g., batimastat, etc.); 17) angiogenesis inhibitors; 18) proteosome inhibitors (e.g., VELCADE); 19) inhibitors of acetylation and/or methylation (e.g., HDAC inhibitors); 20) modulators of NF kappa B; 21) inhibitors of cell cycle regulation (e.g., CDK inhibitors); and 22) modulators of p53 protein function.

As defined in claims 9-11, the pharmaceutical compositions are for use in the treatment of cancer (e.g., PDAC), for example by making the cancer less aggressive, making the cancer/tumor susceptible to other treatments (e.g., chemotherapy), inhibiting metastasis, killing cancer cells, etc.

The pharmaceutical compositions described herein can be used before and/or after surgery to remove a tumor or cancerous tissue. The pharmaceutical compositions described herein can be administered before, during, or after another cancer treatment (e.g., immunotherapy, chemotherapy, etc.). The pharmaceutical compositions described herein may be administered to a subject at risk for cancer (e.g., precancerous, genetic risk factors, environmental risk factors, lifestyle risk factors, etc.) to prevent cancer. The pharmaceutical compositions described herein may be administered to a subject in remission from to prevent the reoccurrence of cancer and/or development of metastasis. The pharmaceutical compositions described herein may be administered to a subject suffering from cancer to kill the cancer cells, reduce tumor size, prevent metastasis, and/or to render the cancer cells susceptible to other treatments.

### EXPERIMENTAL

### IL-22R1 is present murine PDAC cells lines as well as established tumors

To assess the biologic impact of IL-22 on PDAC initiation and progression, an established transgenic murine model of spontaneous autochthonous pancreas cancer was used. The PKCY mouse (ref. 21) is a transgenic Cre-lox model with *Pdx-1* driven expression of mutant KRAS^{G12D} and a heterozygous deletion of p53. These mice reliably develop all stages of PDAC including early and late PanIN (8-10 weeks of life), invasive cancer and metastases (16-20 weeks of life). Tumor lines were established from PKCY mice and immunoblotting and RT-PCR confirmed IL-22R1 expression. Immunohistochemical (IHC) staining of normal mouse pancreas confirmed homogenous expression on acinar cells as has been demonstrated (ref. 14). To assess IL-22R1 on pre-invasive and invasive pancreas lesions, pancreata from PKCY mice at 10 weeks and 20 weeks of age (after ultrasound confirmation of tumor formation) were harvested for IHC. These data confirm robust expression of IL-22R1 in murine pancreatic neoplasms as well as normal pancreas.

### IL-22 is elevated in pancreatitis and PDAC compared to normal pancreas and non-pancreatic tissues

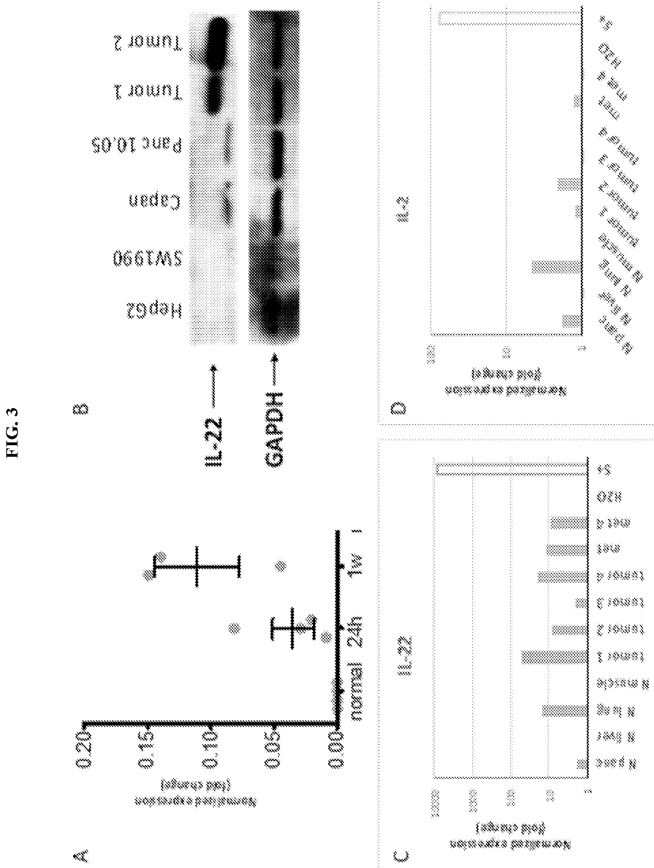

To assess IL-22 in pancreatitis, 8-week-old PKCY mice were given 7x hourly intraperitoneal injections of the cholecystokinin (CCK) analogue cerulean. IL-22, as measured by RT-PCR, was elevated at 24 hours and interestingly was even higher at 7 days, long after the acute inflammatory process is abating (Figure 3A). To quantify the amount of IL-22 present in human tumors, immunoblotting was performed on lysates from surgical specimens. When compared to PDAC cell lines, which produced no IL-22, there was abundant IL-22 in resected pancreatic tumors (Figure 3B). To characterize the amount of IL-22 relative to normal pancreatic tissue and other organs, tissues were harvested from PKCY tumor bearing and wild-type mice. RT-PCR was used to measure relative amounts of IL-22 mRNA present in normal pancreas, PDAC, metastases, lung and liver tissue as compared to background (muscle). The data revealed high levels of IL-22 in pancreatic cancer and metastases relative to normal pancreas and other tissues with the exception of lung (Figure 3C). To ensure this did not simply represent a nonspecific increase in cytokines, IL-2, IL-6, IL-10 and GM-CSF were also measured and unlike IL-22, showed equal or lower levels in tumors compared to normal pancreas (Figure 3D). These experiments confirm that IL-22 is present at elevated levels in both human and murine PDAC as well as pancreatitis.

### IL-22 upregulates STAT3 and NF-κB phosphorylation in PDAC

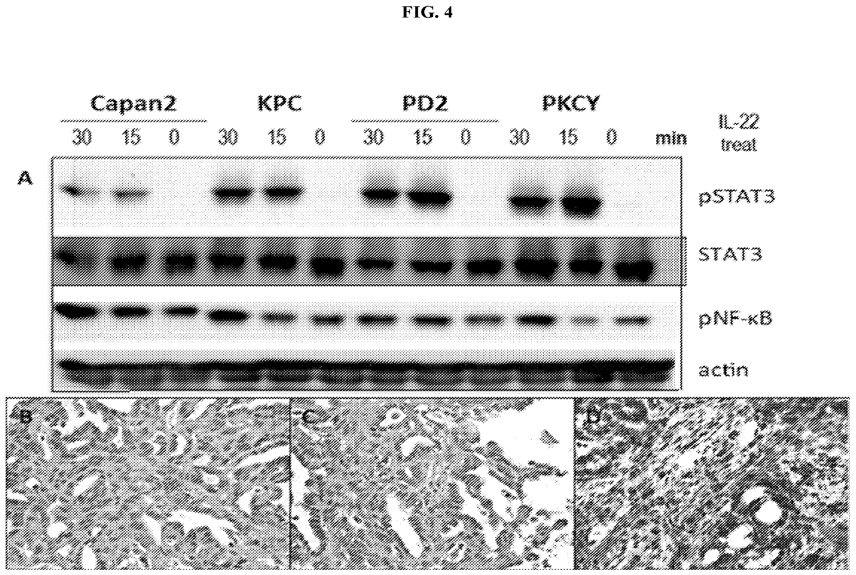

One of the principle signaling pathways activated by ligand binding at IL-22R1 is via JAK mediated phosphorylation of STAT3 (pSTAT3) (Refs. 7, 36, 37). Because of constitutive association of STAT3 with the intracellular domain of the receptor, signaling is activated rapidly and efficiently upon IL-22 binding (ref. 38). A second, less described IL-22 signaling pathway is via NF-κB, which leads to transcription of genes associated with proliferation and prevention of apoptosis (ref. 6, 7). To evaluate the ability of IL-22 to activate STAT3 and NF-κB in human and mouse PDAC, cell lines were incubated with 100ng/ml of IL-22 for 15 and 30 minutes. Immunoblotting revealed robust pSTAT3 activation and modest increase in pNF-κB (KPC and PKCY lines), which was constitutively expressed at baseline in all tested cell lines (Figure 4A). To determine the presence of STAT3 activation in-vivo, IHC for pSTAT3 was performed on pancreata harvested from PKCY mice at 8 weeks (low-grade PanIN), 12 weeks (high-grade PanIN) and after ultrasound confirmation of PDAC. pSTAT3 staining was increased as lesions advanced from low grade PanIN to invasive cancer (Figure 4B-D). These data demonstrate that IL-22 is capable of inducing STAT3 and NF-κB activation in PDAC and that pSTAT3 increases with more aggressive tumor histology.

### IL-22 promotes pancreatic cancer tumorigenesis

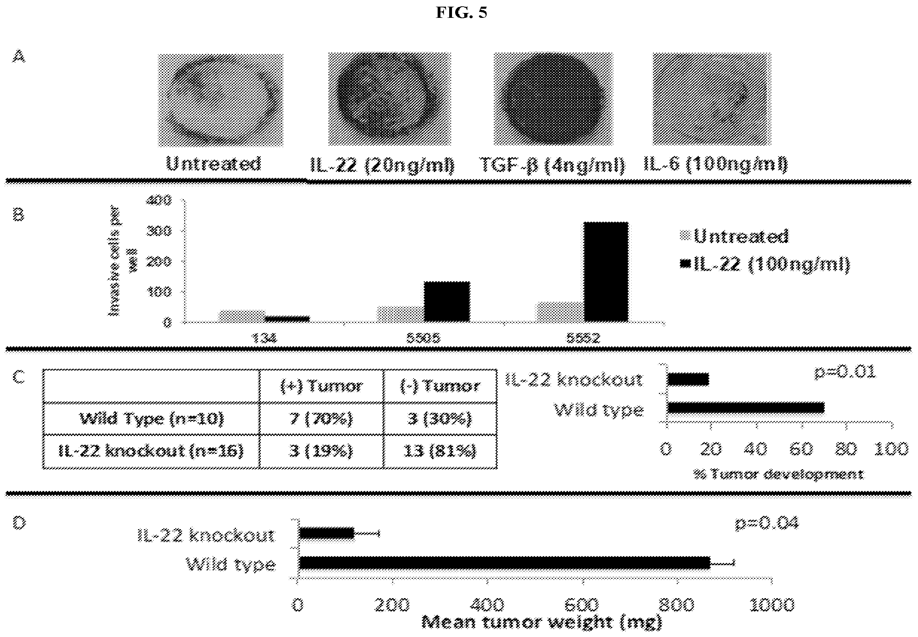

To establish the role of IL-22 in pancreatic tumorigenesis *in vitro,* the murine PDAC line PKCY and PanIN lines 134, 5505 and 5552 were incubated in IL-22. Matrigel invasion assays were performed and number of infiltrating cells quantified. IL-22 incubation induced a 10-fold increase in invasion of PKCY cells compared to control conditions (untreated and IL-6) (Figure 5A). IL-22 induced invasion in two PanIN cell lines, which at baseline show little to no invasive capability (Figure 5B). Wild-type and IL-22 knockout mice were subcutaneously inoculated with equal quantities of PKCY cells. At 21 days, mice were sacrificed and tumor development, size and weight were determined. Wild-type mice showed a significantly higher propensity for tumor development (Figure 5C) and produced larger masses (Figure 5D). These data confirm that IL-22 promotes pancreatic tumorigenesis.

### IL-22 leads to increased PDAC chemoresistance to cisplatin

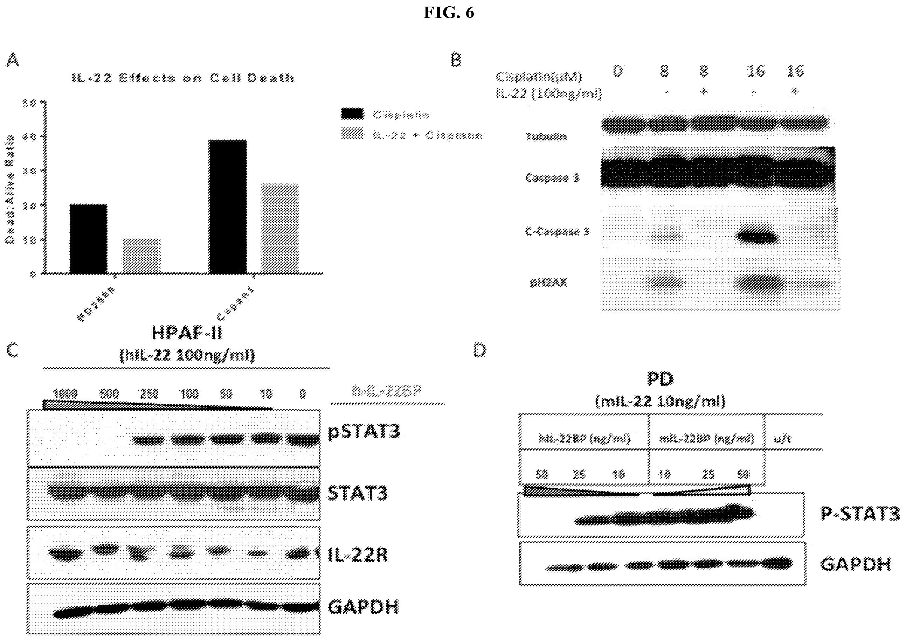

Chemoresistance is a well-established phenomenon in PDAC and is often responsible for disease progression and death. To determine if IL-22 signaling resulted in increased chemoresistance, PDAC cells were cultured in IL-22 for 4 days and phenotypic changes consistent with EMT confirmed by contrast phase microscopy (spindle formation). Cells were then treated with varying doses of cisplatin and cell death assessed by immunoblot for cleaved caspase 3. Degree of DNA damage was determined by measurement of phosphorylated histone 2AX (pH2AX), a marker of DNA damage triggered by cisplatin (ref. 39). The murine PDAC line PD2560 showed sensitivity to cisplatin at low and high doses which was diminished by IL-22 pretreatment (Figure 6A,B). The differences in cell death appeared to be mediated by changes in DNA damage (Figure 6B). These data demonstrate that IL-22 results in increased resistance to the chemotherapeutic cisplatin.

### IL-22BP and a novel fusion protein (IL-22BP-Alb) are capable of sequestering IL-22 and preventing pSTAT3 signaling, in vitro

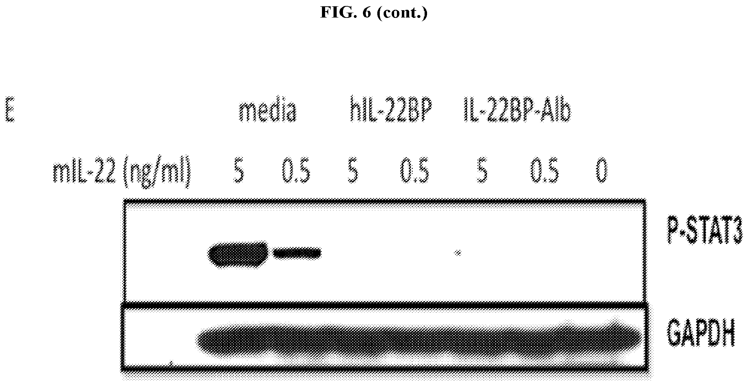

IL-22BP is an important regulator of IL-22 signaling at the tissue level and functions by sequestering cytokine and blocking its ability to bind to its cognate receptor. Its high affinity for IL-22 and neutralizing ability make it an attractive target for pharmacologic disruption of IL-22 signaling. To determine its efficacy *in vitro,* a fixed concentration of human and mouse IL-22 (100ng/ml) was incubated for 30 minutes with increasing concentrations of human or mouse IL-22BP (0-1000ng/ml). The mixture was then placed onto human and murine PDAC lines and IL-22 signaling measured by pSTAT3 activation at 15 minutes. Human IL-22BP was capable of completely neutralizing both murine and human IL-22, while murine IL-22BP showed little sequestration (Figure 6C,D). A fusion protein which binds hIL-22BP to murine albumin was constructed. *In vitro* testing for sequestration and abrogation of STAT3 signaling revealed the IL-22BP-Alb fusion protein was capable of neutralizing IL-22 (Figure 6E). These data confirm the ability to disrupt the IL-22/IL-22R1 axis by cytokine neutralization.

### Abrogation of STAT3 signaling encountered with IL-22BP neutralization translates into reduced EMT and stemness, in vitro

IL-22 functions primarily through STAT3 signaling and other activated pathways, including NF-κB, STAT1 and STAT6. To confirm that IL-22 neutralization by IL-22BP results in decreased pro-tumorigenic factors including EMT and stemness, 10ng/ml of IL-22 are incubated with varying doses of IL-22BP or media alone for 30 minutes. The mixture is added to murine PDAC cells in low attachment plates and incubated for 1, 3, and 7 days. Neutralization is confirmed by immunoblot for pSTAT3, pSTAT1, pSTAT6 and nuclear p65. RNA is collected and qRT-PCR performed to assess upregulation of the EMT transcription factors ZEB1, Slug and Twist. At 7 days sphere formation is determined by light microscopy and the number and size of spheres recorded.

### Determination of the pharmacokinetics (PK) ofIL-22BP, in-vitro, using hepatic microsomes

The liver mediates 60-80% of circulating protein degradation (ref. 40). Hepatic microsomes are subcellular vesicles derived from pooled liver endoplasmic reticula which contain many of the enzymes responsible for metabolism. Incubation of microsomes with drugs or biologic compounds provides an estimate of the anticipated *in vivo* PK. Varying doses of buffered IL-22BP is incubated with pooled murine liver microsomes and NADPH. Samples are collected a 0, 1, 5, 10, 15, 30 and 60 minutes and amount of remaining IL-22BP determined by quantitative ELISA.

### Evaluating the pharmacokinetics (PK) of IL-22BP in mice

To assess the PK of IL-22BP, mixed background 10-week-old mice, randomly assigned to groups by weight, are used. Mice are administered IL-22BP intravenously by tail vain injection (IV), intraperitoneally (IP) and subcutaneously at doses of 1mg/kg, 1mg/kg and 1.5mg/kg, respectively. This dose may be adjusted based on the results of the *in vitro* pharmacokinetic results. Mice are closely monitored for any adverse reactions. At 0.5, 2, 4, 8, 16, 24 and 48 hours, mice are sacrificed and blood and organs collected and placed at -80 °C. Blood is batch thawed and centrifuged to collect serum. IL-22BP is measured by sandwich ELISA and immunoblotting.

### Determination of the effect of IL-22BP administration on pancreatic tumorigenesis in an orthotopic model of pancreas cancer

Experiments demonstrated that IL-22 induces EMT and a stem-like phenotype via STAT3 signaling in murine PDAC cells, *in vitro.* To translate these findings into an *in vivo* system, syngeneic mixed background mice are orthotopically inoculated with pancreas cancer cells (ref. 41). Mice are anesthetized and the left flank incised to enter the peritoneum. The spleen is identified and delivered out of the abdomen with the attached pancreas. A 27-gauge needle is used to deliver 5x10⁵ PDAC cells suspended in 100µL of PBS into the recipient pancreas then the incision is closed and mice are recovered from anesthesia. To test the impact of IL-22BP on tumor establishment, PDAC cells are orthotopically injected in the presence of 100ng/ml of IL-22BP. Pancreata are visualized by ultrasonography three times per week to assess tumor development. Mice are sacrificed at 3 weeks and pancreata weighed and assessed for small tumors by IHC. To determine the impact of IL-22BP on established tumors, orthotopic injection is performed and tumor establishment confirmed by ultrasound. Once tumors are identified, mice are administered PBS or IL-22BP three times per week (with dose and timing adjusted based on the *in vitro* and *in vivo* PK studies). After two weeks, mice are sacrificed and pancreata harvested for analysis. Pancreata are weighed and size measured. Pancreas samples are processed to single cell suspension for analysis of immune cell infiltrate by flow cytometry, and formalin-fixed for IHC staining for pSTAT3 and ZEB1 to determine impact on IL-22 signaling and induction of EMT, Ki-67 to assess differences in cell proliferation, and ALDH1 and CD44 as a measure of stem cell populations (ref. 42).

### Determination the effect of neutralization of IL-22 on sensitivity to cisplatin, in vitro

Experiments demonstrated that IL-22 increases DNA damage repair in PDAC cells rendering them more resistant to cisplatin therapy. To determine if IL-22BP abrogates this *in vitro,* IL-22 is incubated with IL-22BP and the mixture is applied to PDAC cells for 48 hours. Cells are then treated with 16µM and 32µM of cisplatin and cell viability tested by trypan blue exclusion and flow cytometry for annexin and 7-AAD. Immunoblotting for cleaved caspase 3 and pH2AX is used to determine apoptosis and DNA damage, respectively.

### Impact of IL-22 neutralization on cisplatin sensitivity in an orthotopic model of PDAC

10-week-old mice are anesthetized and orthotopic tumors are established. Mice are evaluated 3 times per week by ultrasonography to determine when tumors have formed. After tumors are 1cm in size, mice are randomly assigned to groups and treated with PBS or 1mg/kg of IL-22BP. After 1 week (day 7), mice receive 3 doses of cisplatin (6mg/kg) or PBS on days 7, 9 and 11. Mice are sacrificed on day 12 and tumors measured for size and weight. A portion of the tumor is collected for IHC and H&E slides prepared to assess necrosis and immune infiltration. TUNEL and cleaved caspase staining is performed to assess differences in apoptosis. Staining for pH2AX identifies any difference in DNA damage.

### Identification of IL-22BP mediated changes in cisplatin sensitivity in a spontaneous autochthonous pancreas cancer model

The development of transgenic mice with pancreas specific mutations in KRAS and deleted tumor suppressor p53 has allowed for *in vivo* study of all stages of pancreas cancer from pre-neoplasia to invasion and metastases. PKCY mice are genotyped to confirm mutations in KRAS and heterozygous deletion of p53. At 16 weeks of age, mice are subjected to weekly ultrasonography until tumors are identified. Once a tumor reaches approximately 1cm, they are randomized and treated as above. At necropsy, pancreata are collected and assayed for cisplatin mediated apoptosis and DNA damage.

### IL-22 sequestering fusion protein

IL-22BP is a relatively small protein, approximately 35-45 kilodaltons (kDa) in size (ref. 32), well below the glomerular filtration pore size of the kidney which is estimated to be ~60kDa. As such, the serum half-life of IL-22BP is short. While this limitation is overcome in a research context by frequent dosing of mice with IL-22BP, such frequent dosing is not feasible in a clinical setting. To develop an IL-22-based therapeutic reagent for humans, a fusion protein combining human IL-22BP to mouse or human albumin has been constructed, to increase the biologic half-life of the IL-22BP. Experiments demonstrated that this fusion protein, IL-22BP-Alb, is capable of binding and neutralizing both human and mouse IL-22, *in-vitro* (Figure 6C-E). Albumin deposition is higher within the tumor microenvironment, thereby facilitating IL-22BP delivery specifically to tumor tissues (ref. 45).

### Optimization of fusion protein half-life and efficacy

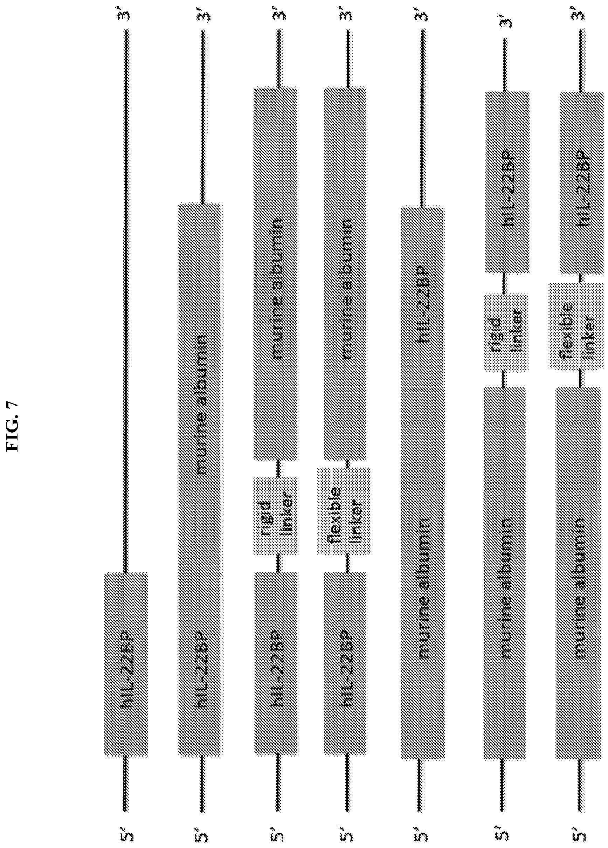

Experiments were conducted to improve the function and stability of fusion proteins. Overhang PCR was used to fuse human IL-22BP, cloned from colitis, to the 5'end of murine albumin, cloned from mouse liver. One strategy to improve functionality of a fusion protein is adding a rigid or flexible linker that can prevent interference. Variants of the IL-22BP-Alb construct have been designed that introduce flexible and rigid linkers and attaching the IL-22BP to the 3' end (Figure 7). Constructs are tested *in vitro* for their ability to bind and neutralized IL-22. Equivalent doses of the fusion proteins are incubated with increasing doses of IL-22.

### Determining the PK of IL-22BP-Alb

cDNA encoding the fusion protein IL-22BP-Alb is expressed in a lentiviral vector and used to transduce 293T cells. After stable expression is confirmed by a reported gene (eGFP), cells are cultured in the absence of serum for 48 hours and the supernatant collected. After centrifugation to clear cell debris, supernatant are serially ultracentrifuged to concentrate proteins then applied to a Q-Sepharose Fast Flow column (GE life science), eluted with a linear gradient from 0 to 1.0 M NaCl in 20 mM PB at a pH 6.0, and analyzed by 12% SDS-PAGE. Fractions containing IL-22BP-Alb are combined and the concentration measured using ELISA for murine albumin (Abcam). In-vitro PK analysis is performed using hepatic microsomes as above and compared to unconjugated IL-22BP. Ten week old mixed background mice are injected IV, IP and SQ with IL-22BP-Alb as above, adjusting the quantity injected to account for the added weight of albumin. At 0.5, 2, 4, 8, 16, 24 and 48 hours, mice are sacrificed and blood and organs collected and placed at -80 °C. Blood is batch thawed and centrifuged to collect serum. IL-22BP is measured by sandwich ELISA and immunoblotting.

### Determination of the effect of IL-22BP-Alb administration on pancreatic tumorigenesis in an orthotopic and spontaneous autochthonous model of pancreas cancer

Orthotopic tumors are established in 10-week-old mice as described above. Mice are evaluated 3 times per week by ultrasonography to determine when tumors have formed. Once tumors are identified, mice are administered PBS, IL-22BP or IL-22BP-Alb three times per week. After two weeks, mice are sacrificed and pancreata harvested, weighed and tumor size measured. A portion of the pancreas is processed to single cell suspension for analysis of immune cell infiltrate by flow cytometry. The remainder is placed in formalin and paraffin embedded for analysis by IHC. Staining is performed for pSTAT3 and ZEB1 to determine impact on IL-22 signaling induction of EMT and Ki-67 to assess differences in cell proliferation. Differences in stem cell populations are determined by staining for ALDH1 and CD44 (ref. 42). To determine efficacy in the spontaneous model of PDAC, PKCY mice are genotyped to confirm mutations in KRAS and deletion of p53. At 16 weeks of age, mice undergo weekly ultrasonography until tumors are identified. Once tumors reach 1cm in diameter, mice are randomized and dosed with PBS, IL-22BP or IL-22BP-Alb three times per week. After two weeks of treatment, pancreata are collected and assayed for STAT3 signaling, EMT activation and stemness as above.

### REFERENCES

The following references, some of which are cited above by number.
1. Whitcomb DC. Inflammation and Cancer V. Chronic pancreatitis and pancreatic cancer. Am J Physiol Gastrointest Liver Physiol 2004; 287(2):G315-9.
2. Farrow B, Sugiyama Y, Chen A, et al. Inflammatory mechanisms contributing to pancreatic cancer development. Ann Surg 2004; 239(6):763-9; discussion 769-71.
3. Lowenfels AB, Maisonneuve P, Cavallini G, et al. Pancreatitis and the risk of pancreatic cancer. International Pancreatitis Study Group. N Engl J Med 1993; 328(20):1433-7.
4. Perusina Lanfranca M, Lin Y, Fang J, et al. Biological and pathological activities of interleukin-22. J Mol Med (Berl) 2016; 94(5):523-34.
5. Sabat R, Ouyang W, Wolk K. Therapeutic opportunities of the IL-22-IL-22R1 system. Nat Rev Drug Discov 2014; 13(1):21-38.
6. Andoh A, Zhang Z, Inatomi O, et al. Interleukin-22, a member of the IL-10 subfamily, induces inflammatory responses in colonic subepithelial myofibroblasts. Gastroenterology 2005; 129(3):969-84.
7. Lim C, Savan R. The role of the IL-22/IL-22R1 axis in cancer. Cytokine Growth Factor Rev 2014; 25(3):257-71.
8. Sertorio M, Hou X, Carmo RF, et al. Interleukin-22 and IL-22 binding protein (IL-22BP) regulate fibrosis and cirrhosis in hepatitis C virus and schistosome infections. Hepatology 2014.
9. Stoy S, Sandahl TD, Dige AK, et al. Highest frequencies of interleukin-22-producing T helper cells in alcoholic hepatitis patients with a favourable short-term course. PLoS One 2013; 8(1):e55101.
10. Jarnicki A, Putoczki T, Ernst M. Stat3: linking inflammation to epithelial cancer - more than a "gut" feeling? Cell Div 2010; 5:14.
11. Haura EB, Zheng Z, Song L, et al. Activated epidermal growth factor receptor-Stat-3 signaling promotes tumor survival in vivo in non-small cell lung cancer. Clin Cancer Res 2005; 11(23):8288-94.
12. Buettner R, Mora LB, Jove R. Activated STAT signaling in human tumors provides novel molecular targets for therapeutic intervention. Clin Cancer Res 2002; 8(4):945-54.
13. Pikarsky E, Porat RM, Stein I, et al. NF-kappaB functions as a tumour promoter in inflammation-associated cancer. Nature 2004; 431(7007):461-6.
14. Aggarwal S, Xie MH, Maruoka M, et al. Acinar cells of the pancreas are a target of interleukin-22. J Interferon Cytokine Res 2001; 21(12):1047-53.
15. Feng D, Park O, Radaeva S, et al. Interleukin-22 ameliorates cerulein-induced pancreatitis in mice by inhibiting the autophagic pathway. Int J Biol Sci 2012; 8(2):249-57.
16. Xue J, Nguyen DT, Habtezion A. Aryl hydrocarbon receptor regulates pancreatic IL-22 production and protects mice from acute pancreatitis. Gastroenterology 2012; 143(6):1670-80.
17. Xu X, Tang Y, Guo S, et al. Increased intratumoral interleukin 22 levels and frequencies of interleukin 22-producing CD4+ T cells correlate with pancreatic cancer progression. Pancreas 2014; 43(3):470-7.
18. Wen Z, Liao Q, Zhao J, et al. High expression of interleukin-22 and its receptor predicts poor prognosis in pancreatic ductal adenocarcinoma. Ann Surg Oncol 2014; 21(1):125-32.
19. Nitecki SS, Sarr MG, Colby TV, et al. Long-term survival after resection for ductal adenocarcinoma of the pancreas. Is it really improving? Ann Surg 1995; 221(1):59-66.
20. Connolly MM, Dawson PJ, Michelassi F, et al. Survival in 1001 patients with carcinoma of the pancreas. Ann Surg 1987; 206(3):366-73.
21. Rhim AD, Mirek ET, Aiello NM, et al. EMT and dissemination precede pancreatic tumor formation. Cell 2012; 148(1-2):349-61.
22. Guerra C, Schuhmacher AJ, Canamero M, et al. Chronic pancreatitis is essential for induction of pancreatic ductal adenocarcinoma by K-Ras oncogenes in adult mice. Cancer Cell 2007; 11(3):291-302.
23. Clark CE, Hingorani SR, Mick R, et al. Dynamics of the immune reaction to pancreatic cancer from inception to invasion. Cancer Res 2007; 67(19):9518-27.
24. Lesina M, Kurkowski MU, Ludes K, et al. Stat3/Socs3 activation by IL-6 transsignaling promotes progression of pancreatic intraepithelial neoplasia and development of pancreatic cancer. Cancer Cell 2011; 19(4):456-69.
25. Takamori H, Oades ZG, Hoch OC, et al. Autocrine growth effect of IL-8 and GROalpha on a human pancreatic cancer cell line, Capan-1. Pancreas 2000; 21(1):52-6.
26. Kuwada Y, Sasaki T, Morinaka K, et al. Potential involvement of IL-8 and its receptors in the invasiveness of pancreatic cancer cells. Int J Oncol 2003; 22(4):765-71.
27. Aujla SJ, Kolls JK. IL-22: a critical mediator in mucosal host defense. J Mol Med (Berl) 2009; 87(5):451-4.
28. Cella M, Fuchs A, Vermi W, et al. A human natural killer cell subset provides an innate source of IL-22 for mucosal immunity. Nature 2009; 457(7230):722-5.
29. Kirchberger S, Royston DJ, Boulard O, et al. Innate lymphoid cells sustain colon cancer through production of interleukin-22 in a mouse model. J Exp Med 2013; 210(5):917-31.
30. Kryczek I, Lin Y, Nagarsheth N, et al. IL-22(+)CD4(+) T cells promote colorectal cancer stemness via STAT3 transcription factor activation and induction of the methyltransferase DOT1L. Immunity 2014; 40(5):772-84.
31. Kalluri R, Weinberg RA. The basics of epithelial-mesenchymal transition. J Clin Invest 2009; 119(6):1420-8.
32. Huber S, Gagliani N, Zenewicz LA, et al. IL-22BP is regulated by the inflammasome and modulates tumorigenesis in the intestine. Nature 2012; 491(7423):259-63.
33. Jones BC, Logsdon NJ, Walter MR. Structure of IL-22 bound to its high-affinity IL-22R1 chain. Structure 2008; 16(9):1333-44.
34. Shah AN, Summy JM, Zhang J, et al. Development and characterization of gemcitabine-resistant pancreatic tumor cells. Ann Surg Oncol 2007; 14(12):3629-37.
35. Wang Z, Li Y, Ahmad A, et al. Pancreatic cancer: understanding and overcoming chemoresistance. Nat Rev Gastroenterol Hepatol 2011; 8(1):27-33.
36. Pickert G, Neufert C, Leppkes M, et al. STAT3 links IL-22 signaling in intestinal epithelial cells to mucosal wound healing. J Exp Med 2009; 206(7):1465-72.
37. Lejeune D, Dumoutier L, Constantinescu S, et al. Interleukin-22 (IL-22) activates the JAK/STAT, ERK, JNK, and p38 MAP kinase pathways in a rat hepatoma cell line. Pathways that are shared with and distinct from IL-10. J Biol Chem 2002; 277(37):33676-82.
38. Dumoutier L, de Meester C, Tavernier J, et al. New activation modus of STAT3: a tyrosine-less region of the interleukin-22 receptor recruits STAT3 by interacting with its coiled-coil domain. J Biol Chem 2009; 284(39):26377-84.
39. Clingen PH, Wu JY, Miller J, et al. Histone H2AX phosphorylation as a molecular pharmacological marker for DNA interstrand crosslink cancer chemotherapy. Biochem Pharmacol 2008; 76(1):19-27.
40. Gebhardt R, Hengstler JG, Muller D, et al. New hepatocyte in vitro systems for drug metabolism: metabolic capacity and recommendations for application in basic research and drug development, standard operation procedures. Drug Metab Rev 2003; 35(2-3):145-213.
41. Kim MP, Evans DB, Wang H, et al. Generation of orthotopic and heterotopic human pancreatic cancer xenografts in immunodeficient mice. Nat Protoc 2009; 4(11):1670-80.
42. Mizukami T, Kamachi H, Mitsuhashi T, et al. Immunohistochemical analysis of cancer stem cell markers in pancreatic adenocarcinoma patients after neoadjuvant chemoradiotherapy. BMC Cancer 2014; 14:687.
43. Liu M, Huang Y, Hu L, et al. Selective delivery of interleukine-1 receptor antagonist to inflamed joint by albumin fusion. BMC Biotechnol 2012; 12:68.
44. Siddik ZH. Cisplatin: mode of cytotoxic action and molecular basis of resistance. Oncogene 2003; 22(47):7265-79.
45. Rogers B, Dong D, Li Z, et al. Recombinant human serum albumin fusion proteins and novel applications in drug delivery and therapy. Curr Pharm Des 2015; 21(14):1899-907.
46. Burgess-Brown NA, Sharma S, Sobott F, et al. Codon optimization can improve expression of human genes in Escherichia coli: A multi-gene study. Protein Expr Purif 2008; 59(1):94-102.

### SEQUENCES

SEQ ID NO: 1 (IL-22BP; Homo sapiens) 
MMPKHCFLGFLISFFLTGVAGTQSTHESLKPQRVQFQSRNFHNILQWQPGRALTGNSSV
YFVQYKIYGQRQWKNKEDCWGTQELSCDLTSETSDIQEPYYGRVRAASAGSYSEWSMT
PRFTPWWETKIDPPVMNITQVNGSLLVILHAPNLPYRYQKEKNVSIEDYYELLYRVFIIN
NSLEKEQKVYEGAHRAVEIEALTPHSSYCVVAEIYQPMLDRRSQRSEERCVEIP
SEQ ID NO: 2 (IL-22A2; Mus musculus) 
MMPKHCLLGLLIILLSSATALEIQPARVSLTPQKVRFQSRNFHNILHWQAGSSLPSNNSIY
FVQYKMYGQSQWEDKVDCWGTTALFCDLTNETLDPYELYYGRVMTACAGRHSAWTR
TPRFTPWWETKLDPPVVTITRVNASLRVLLRPPELPNRNQSGKNASMETYYGLVYRVFT
INNSLEKEQKAYEGTQRAVEIEGLIPHSSYCVVAEMYQPMFDRRSPRSKERCVQIP
SEQ ID NO: 3 (IL-22BP; consensus) 
MMPKHCXLGXLIXXXXXXAXXXQXXXXSLXPQXVXFQSRNFHNILXWQXGXXLXX
NXSXYFVQYKXYGQXQWXXKXDCWGTXXLXCDLTXETXDXXEXYYGRVXXAXAGX
XSXWXXTPRFTPWWETKXDPPVXXITXVNXSLXVXLXXPXLPXRXQXXKNXSXEXYY
XLXYRVFXINNSLEKEQKXYEGXXRAVEIEXLXPHSSYCVVAEXYQPMXDRRSXRSXE
RCVXIP
 X = any residue
SEQ ID NO: 4 (Albumin; Homo sapiens)
SEQ ID NO: 5 (rigid linker; artificial)
   PAPAP
SEQ ID NO: 6 (flexible linker; artificial)
   GGGGSGGGGSGGGGS

## Claims

1. A fusion protein comprising an IL-22BP polypeptide and an albumin.

2. The fusion protein of claim 1, wherein the IL-22BP polypeptide has at least 70% sequence identity with one or more of SEQ ID NOS: 1-2.

3. The fusion protein of claim 1, wherein the fusion exhibits enhanced serum half-life relative to full-length wild-type IL-22BP.

4. The fusion protein of claim 1, wherein the fusion is at least twice the molecular weight of full-length wild-type IL-22BP.

5. The fusion protein of claim 1, wherein the albumin comprises at least 70% sequence identity to SEQ ID NO: 4 or a portion thereof.

6. The fusion protein of claim 1, wherein the IL-22BP polypeptide and the albumin are connected directly, by a peptide linker, or by a non-peptide linker.

7. A pharmaceutical composition comprising the fusion protein of one of claims 1-6 and a pharmaceutically acceptable carrier.

8. Pharmaceutical composition of claim 7 for use in the treatment or prevention of a disease.

9. Pharmaceutical composition for use of claim 8, wherein the disease is cancer.

10. Pharmaceutical composition for use of claim 9, wherein the disease is pancreatic cancer.

11. Pharmaceutical composition for use of claim 10, wherein the disease is pancreas adenocarcinoma (PDAC).

## Patentansprüche

1. Fusionsprotein, umfassend ein IL-22BP-Polypeptid und ein Albumin.

2. Fusionsprotein nach Anspruch 1, wobei das IL-22BP-Polypeptid mindestens 70 % Sequenzidentität mit einer oder mehreren der SEQ ID NOs: 1-2 aufweist.

3. Fusionsprotein nach Anspruch 1, wobei die Fusion eine erhöhte Serumhalbwertszeit im Vergleich zu Wildtyp-IL-22BP voller Länge aufweist.

4. Fusionsprotein nach Anspruch 1, wobei die Fusion mindestens das doppelte Molekulargewicht von Wildtyp-IL-22BP voller Länge aufweist.

5. Fusionsprotein nach Anspruch 1, wobei das Albumin mindestens 70 % Sequenzidentität mit SEQ ID NO: 4 oder einem Teil davon aufweist.

6. Fusionsprotein nach Anspruch 1, wobei das IL-22BP-Polypeptid und das Albumin direkt, durch einen Peptidlinker oder durch einen Nicht-Peptidlinker verbunden sind.

7. Pharmazeutische Zusammensetzung, umfassend das Fusionsprotein nach einem der Ansprüche 1 bis 6 und einen pharmazeutisch akzeptablen Träger.

8. Pharmazeutische Zusammensetzung nach Anspruch 7 zur Verwendung bei der Behandlung oder Vorbeugung einer Krankheit.

9. Pharmazeutische Zusammensetzung zur Verwendung nach Anspruch 8, wobei die Krankheit Krebs ist.

10. Pharmazeutische Zusammensetzung zur Verwendung nach Anspruch 9, wobei die Krankheit Bauchspeicheldrüsenkrebs ist.

11. Pharmazeutische Zusammensetzung zur Verwendung nach Anspruch 10, wobei die Krankheit ein Pankreas-Adenokarzinom (PDAC) ist.

## Revendications

1. Protéine de fusion comprenant un polypeptide d'IL-22BP et une albumine.

2. Protéine de fusion selon la revendication 1, dans laquelle le polypeptide d'IL-22BP a au moins 70 % d'identité de séquence avec une ou plusieurs des SEQ ID NO: 1-2.

3. Protéine de fusion selon la revendication 1, dans laquelle la fusion présente une demi-vie sérique améliorée par rapport à l'IL-22BP de type sauvage de pleine longueur.

4. Protéine de fusion selon la revendication 1, dans laquelle la fusion a au moins deux fois le poids moléculaire de l'IL-22BP de type sauvage de pleine longueur.

5. Protéine de fusion selon la revendication 1, dans laquelle l'albumine comprend au moins 70 % d'identité de séquence avec la SEQ ID NO: 4 ou une partie de celle-ci.

6. Protéine de fusion selon la revendication 1, dans laquelle le polypeptide d'IL-22BP et l'albumine sont reliés directement, par un lieur peptidique ou par un lieur non peptidique.

7. Composition pharmaceutique comprenant la protéine de fusion selon l'une des revendications 1-6 et un support pharmaceutiquement acceptable.

8. Composition pharmaceutique selon la revendication 7, pour utilisation dans le traitement ou la prévention d'une maladie.

9. Composition pharmaceutique pour utilisation selon la revendication 8, dans laquelle la maladie est le cancer.

10. Composition pharmaceutique pour utilisation selon la revendication 9, dans laquelle la maladie est le cancer du pancréas.

11. Composition pharmaceutique pour utilisation selon la revendication 10, dans laquelle la maladie est l'adénocarcinome du pancréas (PDAC).
